# EUROPÄISCHE PATENTSCHRIFT

(11) **EP 0 901 337 B1**
(45) Veröffentlichungstag und Bekanntmachung des Hinweises auf die Patenterteilung: **06.09.2000**
(21) Anmeldenummer: 97923826.8
(22) Anmeldetag: 13.05.1997
(51) Int. Cl.: A61B 3/028

(54) **ELEKTROMOTORISCH BETRIEBENER PHOROPTER**
ELECTROMOTIVE DRIVEN PHOROPTER
PHOROPTRE A ENTRAINEMENT ELECTROMOTEUR

(30) Priorität: 21.05.1996 DE 19620326
(43) Veröffentlichungstag der Anmeldung: 17.03.1999
(73) Patentinhaber: PREUSSNER, Paul Rolf, Dr., 55131 Mainz (DE)
(72) Erfinder: PREUSSNER, Paul Rolf, Dr., 55131 Mainz (DE)
(86) Internationale Anmeldenummer: DE9700989
(87) Internationale Veröffentlichungsnummer: WO9743945

(56) Entgegenhaltungen:
- DE-A- 3 331 799
- DE-A- 4 124 056
- US-A- 4 500 180
- US-A- 4 596 449

## Beschreibung

### Technisches Gebiet

Bei der Erfindung handelt es sich um ein optomechanisches Gerät, des Augenarztes oder Optikers, mit dem die Testgläser im Rahmen der subjektiven Anpassung von Korrekturgläsern variiert werden können.

### Stand der Technik

Im Rahmen der subjektiven Anpassung von Korrekturgläsern (Brille oder Kontaktlinsen) wird für das Vorsetzen von Testgläsern heute i.a. ein sogenannter "Phoropter" verwendet. In Geräten, die dem Stand der Technik entsprechen (z.B. US4500180, DE2901459), sind dabei sphärische und zylindrische Linsen für jedes Auge meist auf Linsenscheiben (Revolverrädern) angeordnet. Andere Ausführungen (z.B.DE3331799) verwenden für die Zylindergläser ein Paar von Stokes-Linsen, wie sie seit der Mitte des 19. Jahrhunderts bekannt sind. Der mechanische Aufbau ist mit diesen Stokes-Linsen wesentlich einfacher, da insgesamt nur zwei Linsen für alle Zylinderstärken und -Achsen erforderlich sind, es werden jedoch recht hohe Anforderungen an die Einstellgenauigkeit für die Drehung der beiden Linsen gegeneinander gestellt, und der Zusammenhang zwischen Relativwinkel unnd resultierender Zylinderstärke ist nicht linear. Praktisch realisierbar ist daher ein Paar von Stokes-Linsen in einem Phoropter nur zusammen mit einem Computer, der in jedem Schritt für die gewünschte Zylinderstärke den benötigten Relativwinkel ausrechnet, und der für jede Linse je einen elektromotorischen Antrieb steuert, der eine ausreichende Einstellgenauigkeit gewährleistet.

Generell werden für Phoropterantriebe, sei es für die Revolverräder mit oder ohne Planetengetriebe oder für Stokes-Linsen, heute meist Schrittmotoren verwendet, wie sie dem Stand der Technik entsprechen. Diese Schrittmotoren treiben dann mit geeigneten mechanischen Untersetzungen die Fassungen der optischen komponenten an, was meist einen nicht unerheblichen Aufwand mit entsprechenden Störungsmöglichkeiten und Herstellungskosten bedeutet. Außerdem sind die mechanischen Abmessungen solcher Phoropter recht groß.

In neuerer Zeit sind auf dem Markt auch Phoropter verfügbar (DE4425443), die anstelle der genannten Schrittmotorantriebe elektrostriktive (i.a. piezoelektrische) Mikro-Schrittmotoren verwenden. Mit diesen Antrieben ist der mechanische Aufwand deutlich geringer, und auch die Positioniergenauigkeit (Schrittzahlen pro Umdrehung) ist größer. Allerdings weisen diese Antriebe meist einen gewissen "Schlupf" auf, so daß zusätzliche Maßnahmen zur ständigen Positionsmessung erforderlich sind. Außerdem müssen sehr enge Fertigungstoleranzen eingehalten werden, was die Kosten zusätzlich erhöht.

**Aufgabe der vorliegenden Erfindung** ist es, einen Phoropter mit einem elektromechanischen Antrieb verfügbar zu machen, der eine sehr hohe Einstellgenauigkeit mit sehr kleinen mechanischer Abmessungen und geringem Her stellungsaufwand verbindet.

### Kurze Darstellung der Erfindung

Die Aufgabe wird erfindungsgemäß entsprechend den kennzeichnenden Merkmalen des Anspruchs 1 gelöst. Zum Bewegen der rotierenden Komponenten (Revolverräder oder Stokes-Linsen, im folgenden "Rotoren" genannt. während feststehende Antriebsteile zusammenfassend "Statoren" genannt werden) wird ein elektromagnetischer Direktantrieb verwendet, wie in anderem Zusammenhang beispielsweise aus US4596449 bekannt, der folgendermaßen realisiert ist. Auf einem Kreisumfang des jeweiliger Rotors befinden sich *n* magnetisierbare, gleichartige Komponenten (im folgenden "Joche" genannt) in konstanten Winkelabständen α, befestigt in nicht magnetisierbarer Umgebung. Diesen unmittelbar gegenüber auf einem Kreisumfang des Stators befinden sich *m* baugleiche Elektromagnete in ebenfalls konstanten Winkelabständen β. Die Mittelpunkte der beiden genannten Kreisumfänge liegen auf der Drehachse, und die sonstige Geometrie ist so ausgelegt, daß sich Elektromagnete und Joche nur durch einen geringen Luftspalt getrennt gegenüberstehen. Die Winkelabstände α und β sind so gewählt, daß sich eine "Noniusanordnung" für die Positionen der Joche und der Elektromagnete zueinander ergibt, das heißt, daß für den Differenzwinkel γ = |α - β| entweder *m* · γ = α und (*m* + 1) · γ = β oder *n* · γ = β und (*n* + 1) · γ = α erfüllt ist. Wenn nun als Ausgangsposition ein Joch genau einem Elektromagneten gegenübersteht, so kann der Rotor um den Winkel γ weiterbewegt werden, indem der Nachbar des genannten Elektromagneten eingeschaltet wird. Wenn α und β sich nur wenig voneinander unterscheiden, lassen sich reproduzierbar sowie schlupf- und reibungsfrei sehr kleine Verstellwinkel 7 erreichen, ohne daß irgendeine mechanische Untersetzung nötig wäre.

### Bevorzugte Ausführung und Ausführungsalternativen

Der Phoropter besteht in üblicher Bauweise aus zwei gleichartigen, in ihrem Abstand zueinander entsprechend dem Augenabstand des zu Untersuchenden einstellbaren Untereinheiten. In jeder dieser Untereinheiten befinden sich mehrere Revolverscheiben für die sphärischen Gläser und für die Hilfsgläser (Polarisationsfilter, Blenden, Verschluß etc.) in bekannter Technik. Die genannter Revolverscheiben rotieren um eine gemeinsame Achse. In der optischen Achse des zu refraktionierenden Auges befindet sich außerdem ein Paar von Stokes-Linsen für die Zylindergläser.

Zwar können in einer anderen Ausführung der Erfindung auch für die Zylindergläser Revolverräder, und, zum gemeinsamen Verstellen der Zylinderachsen eines Revolverrades, je ein Planetengetriebe in bekannter Technik eingesetzt werden, dabei kommen aber die Vorteile der Erfindung (geringerer mechanischer Aufwand) viel weniger zur Geltung.

Optional kann der Phoropter zusätzlich noch mit einem Paar von Prismenkompensatoren ausgestattet sein, wie sie dem Stand der Technik entsprechen.

Da die Revolverscheiben normalerweise volle Umdrehungen ausführen sollen und in *k* Positionen Linsen oder andere optische Elemente tragen, muß die Anzahl der Joche auf ihnen durch *k* teilbar sein. Eine Revolverscheibe mit sechs Positionen könnte also beispielsweise mit 30 Jochen im Winkelabstand α = 12° bestückt sein. Um sie anzutreiben, genügen drei Elektromagnete im Winkelabstand β = 16°, damit die o.g. "Noniusbedingung" erfüllt ist. Abwechelndes Einschalten der Elektromagnete in der Reihenfolge 1-2-3-1-2-3... führt dann zu einer dauernden Rotation, die solange fortgesetzt wird, bis die gewünschte neue Position erreicht ist.

Um die Einzellinsen eines Paares von Stokes-Linsen so zu drehen, daß die für den genannten Zweck geforderten Abstufungsschritte in der resultierenden Zylinderstärke (normalerweise 0.25dpt) mit ausreichender Genauigkeit (besser als 0.1dpt) bei gleichzeitigem Einstellbereich von mindestens 6.0dpt in der resultierenden Zylinderstärke realisiert werden können, muß der Verstellwinkel für die Einzellinse γ ≤ 1° sein. Dieses wird beispielsweise erreicht, indem auf dem Rotor (Stokes-Linse) 25 Joche im Abstand α = 14.4° und auf dem zugehörigen Stator 16 Elektromagnete im Abstand β = 15.3° aufgebracht werden. Dann ist der Verstellwinkel γ = 0.9°.

Die Ansteuerung der Elektromagnete erfolgt vorzugsweise über einen Computer in der Weise, daß in einem Computerwort jeweils ein Bit für einen Elektromagneten gesetzt wird, wenn dieser Magnet eingeschaltet sein soll. Über entsprechende Computerausgänge können dann beispielsweise Halbleiterschalter eine Konstantstromquelle auf den jeweiligen Magneten schalten. Da fiir jeden Antrieb nur ein Elektromagnet zu einem Zeitpunkt aktiv ist, kann die Zahl der Leitungen zwischen Computer und Phoropter durch elektronische Multiplexer- und Demultiplexerbausteine, wie sie dem Stand der Technik entsprechen, stark reduziert werden.

Die Joche und die Elektromagnete müssen bezüglich ihrer Geometrie, ihres Materials, des Luftspaltes zwischen ihnen sowie des durch den Elektromagneten fließenden Stromes entsprechend den bekannten Regeln der Elektrotechnik so ausgelegt sein, daß ein Elektromagnet, wenn er elektrisch eingeschaltet ist, auf das ihm am nächsten liegende Joch, d.h. auf ein Joch, das höchstens einen Winkelabstand von α/2 zu ihm hat, eine Kraft ausübt, die den Rotor drehen kann, d.h., die mindestens seine Lagerreibung überwindet.

Vorzugsweise wird ein Elektromagnet durch zwei nebeneinander angeordnete Spulen realisiert, die auf der dem Rotor abgewandten Seite des Elektromagneten durch ein weiteres Joch mechanisch und magnetisch miteinander verbunden sind, denn so wird eine geringe Bauhöhe gleichzeitig mit einer hohen Gesamtwindungszahl für die Spulen erreicht.

Mit dem angegebenen "Noniusprinzip" ist bei vorgegebener Abfolge des Einschaltens der Elektromagnete die Position des Rotors nur dann eindeutig, wenn die Ausgangsposition feststeht. Ansonsten ist die (stabile) Rotorposition für einen eingeschalteten Elektromagneten *n*-fach vieldeutig. Aus diesen Gründen müssen Positionsmeßmittel vorgesehen werden, um einen definierten Ausgangszustand zu erreichen. Hierfür können beispielsweise Gabel- oder Reflektionslichtschranken in einer dem Stand der Technik entsprechenden Ausführung verwendet werden. Ihre Winkelauflösung darf deutlich schlechter sein als die mit dem "Noniusantrieb" erreichbare Positionierungsgenauigkeit γ des Rotors. Theoretisch genügt eine Auflösung besser als α/2, mit der eine ein zige Markierung auf einem Umfangskreis des Rotors erkannt werden muß. Bei nicht bekannter Rotorposition wird dann ein Start- bzw. Resetvorgang folgendermaßen durchgeführt. Zunächst werden die Elektromagnete zyklisch nacheinander geschaltet. Bei jedem Schritt wird abgefragt, oh das Positionsmeßmittel die Marke erkannt hat. Ist dies der Fall, so wird nur noch ein als "Nr. 1" bezeichneter Elektromagnet eingeschaltet, so daß der Rotor nur noch um den Winkel rotiert, den das dem Magneten Nr. 1 am nächsten gelegene Joch entfernt ist. Stehen beide übereinander, so ist die gewünschte Startposition erreicht. Jede neue Position ist, ausgehend von dieser Startposition, eindeutig nur durch die Schaltabfolge der Elektromagneten einstellbar. Ein neuer Resetvorgang ist daher erst nach dem Aus- und wieder Einschalten oder nach Auftreten eines Fehlers erforderlich. Bezüglich der Anordnung von Elektromagneten und Jochen, speziell auch bezüglich der Orientierung des Magnetfeldes zwischen ihnen (parallel oder senkrecht oder auch in anderen Winkeln zur Rotorachse) sind zahlreiche Varianten möglich, die hauptsächlich von der gewünschten Gehäuseform oder von fertigungstechnischen Gesichtspunkten abhängen.

Eine besonders einfache und fertigungstechnisch vorteilhafte Konstruktion ergibt sich, wenn die Elektromagnete fiir einen oder auch fiir zwei benachbarte Rotoren auf eine Elektronikplatine gesetzt werden, auf der auch ihre Ansteuerelektronik (Halbleiterschalter, evtl. Demultiplexer) montiert ist.

Zum Betrieb des Phoropters können die Sollwertvorgaben für die Gläser entweder von einer manuell-elektronischen Bedieneinheit, oder von einer automatischen Refraktionseinrichtung, wie etwa aus DE4124056 bekannt, oder von einer Kombination von beidem, die alternativ manuelles oder automatisches Refraktionieren gestattet, übernommen werden. Hierzu müssen dann elektronische Schnittstellen vorgesehen werden, wie sie dem Stand der Technik entsprechen.

Beim Betrieb mit einer manuell-elektronischen Bedieneinheit können dann auch, wie beispielsweise aus DE2901459 bekannt, mit elektronischen Mitteln solche Werte für die Zylinderstärke oder -Achse berechnet und eingestellt werden, wie sie bei manuellen Phoroptern mit Hilfe von Kreuzzylindergläsern realisiert werden.

Wenn die Anzahlen der Joche *n* und der Elektromagnete *m* nicht teilerfremd sind, so sind erfindungsentsprechende Antriebe konstruierbar, bei denen die oben genannte Noniusbedingung sich auf ganzzahlige Teile von *n* und / oder *m* bezieht.

## Patentansprüche

1. Elektromotorisch betriebener Phoropter mit
• mindestens einem drehbaren optischen Element (Rotor), durch dessen Drehung die optische Wirkung verändert wird,
• einem elektromotorischen Antrieb zum Drehen des optischen Elementes,
• einer elektronischen Steuereinrichtung zum manuellen oder programmgesteuerten Einstellen der jeweiligen optischen Wirkung,
**dadurch gekennzeichnet,** daß der elektromotorische Antrieb als Direktantrieb im Sinn eines Schrittmotors wie folgt realisiert ist:
• *n* magnetisierbare, baugleiche Joche sind auf dem Rotor auf einem Kreisumfang in konstanten Winkelabständen α in nicht magnetisierbarer Umgebung befestigt, wobei die Kreisnormale die Drehachse des Rotors ist;
• *m* baugleiche Elektromagnete sind auf einem dem Rotor gegenüberliegenden, ortsfesten Stator in konstanten Winkelabständen β in nicht magnetisierbarer Umgebung auf einem Kreisumfang befestigt, wobei die Kreisnormale die Drehachse des Rotors ist;
• die Anordnung ist so getroffen, daß jeweils ein erregter Elektromagnet ein Joch, das weniger als α/2 von ihm entfernt ist, anzieht und dadurch den Rotor dreht;
• die Winkel α und β sind so gewählt, daß sie eine "Noniusbedingung" erfüllen, das heißt, für den Winkel γ = |α- β| gilt entweder *m*·γ = α und (*m +* 1) · γ = β oder *n* · γ = β und (*n* + 1) · γ = α, wobei *n* und *m* teilerfremd sind, oder es gilt eine entsprechende Bedingung für eine ganzzahliche Teilmenge von *n* und / oder *m*;
• eine elektronische Schaltungseinrichtung aktiviert die Elektromagnete so, daß das optische Element sich entsprechend den Vorgaben der Steuereinrichtung bewegt.

2. Phoropter nach Anspruch 1, **gekennzeichnet dadurch,** daß für den Rotor ein Positionsmeßmittel, beispielsweise eine Lichtschranke, vorgesehen ist, das mindestens eine Position des Rotors mit mindestens einer Winkelauflösung von α/2 erkennt.

3. Phoropter nach Anspruch 1, **dadurch gekennzeichnet,** daß als optische Elemente Revolverscheiben zur Aufnahme von Linsen oder sonstigen optischen Komponenten eingebaut sind, wobei durch Drehung der Revolverscheibe jeweils eine andere Komponente in die optische Achse gefahren wird.

4. Phoropter nach Anspruch 1, **dadurch gekennzeichnet,** daß sich als optische Elemente Stokes-Linsen in der optischen Achse befinden und um diese drehbar sind.

5. Phoropter nach Anspruch 1, **dadurch gekennzeichnet,** daß sich als optische Elemente Prismen in der optischen Achse befinden und um diese drehbar sind.

6. Phoropter nach Anspruch 1, **gekennzeichnet dadurch,** daß die Elektromagnete für einen Rotor oder für zwei benachbarte Rotoren zusammen mit ihrer Ansteuerelektronik auf einer Baueinheit (Platine) montiert sind.

7. Phoropter nach Anspruch 1, **gekennzeichnet dadurch,** daß eine elektronische Schnittstelle vorgesehen ist, über die die Sollwerte für die Phoroptereinstellungen von einer manuell-elektronischen Bedieneinheit, und / oder von einer automatischen Refraktionseinrichtung vorgegeben werden.

8. Phoropter nach Anspruch 4, **gekennzeichnet dadurch,** daß eine manuell-elektronische Bedieneinheit vorgesehen ist, mit der der Bediener die Zylinderstärke oder Zylinderachse von Vorsatzgläsern in für ihn scheinbar der gleichen Weise wie durch den bekannten Kreuzzylinder verändern kann. wobei aber tatsächlich elektronische Mittel vorgesehen sind, die diese Veränderung der Vorsatzgläser durch Überlagerung mit diesem scheinbaren Kreuzzylinder berechnen und die Stokes-Linsen entsprechend ansteuern.

## Claims

1. Electromotive driven phoropter with
• at least one rotatable optical element (rotor) through the rotation of which the optical effect is changed,
• an electromotive drive to rotate the optical element,
• an electronic control device for the manual or program-controlled setting of the respective optical effect,
**characterised by the fact** that the electromotive drive is implemented as a direct drive in the sense of a stepper motor as follows:
• *n* magnetisable, structurally identical yokes are fastened on the rotor on a circumference periphery at constant angle distances α in non-magnetisable surroundings whereby the circle normal line is the rotating axis of the rotor;
• *m* structurally identical electromagnets are fastened on a stationary stator located opposite the rotor at constant angle distances β in non-magnetisable surroundings on a circumference periphery whereby the circle normal line is the rotating axis of the rotor;
• the arrangement is made in such a manner that one actuated electromagnet in each case attracts a yoke that is less than α/2 distant from it, thus driving the rotor;
• the angles α and β are selected in such a manner that they fulfil a "vernier condition", that means for angle γ = |α - β| either *m · γ =* α and (*m* + 1) · γ = β or *n* · γ = βand (*n* + 1) · γ = α apply, whereby *n* and m are relatively prime, or a corresponding condition applies for an integral fraction of *n* and / or *m;*
• an electronic switching device activates the electromagnets in such a manner that the optical element moves in accordance with the settings of the control device.

2. Phoropter in accordance with claim 1, **characterised by the fact** that a position measuring device, for example a light barrier, is provided for the rotor, which recognises at least one position of the rotor with an angle resolution of at least α/2.

3. Phoropter in accordance with claim 1, **characterised by the fact** that revolving discs are fitted as optical elements for the incorporation of lenses or other optical components, whereby by rotating the revolving disc another component is moved into the optical axis in each case.

4. Phoropter in accordance with claim 1, **characterised by the fact** that Stokes' lenses are present in the optical axis as optical elements and are rotatable around the same.

5. Phoropter in accordance with claim 1, **characterised by the fact** that prisms are present in the optical axis as optical elements and are rotatable around the same.

6. Phoropter in accordance with claim 1, **characterised by the fact** that the electromagnets for one rotor or for two adjoining rotors are mounted together with their actuating electronics on a module (pc board).

7. Phoropter in accordance with claim 1, **characterised by the fact** that an electronic interface is provided via which the set values for the phoropter settings are given by a manual-electronic operating unit and/or by an automatic refracting device.

8. Phoropter in accordance with claim 4, **characterised by the fact** that a manual-electronic operating unit is provided with which the operator can change the cylinder strength or cylinder axis of supplementary lenses in apparently the same manner for him as by the known cross cylinder, whereby, however, in fact electronic means are provided which calculate this change in the supplementary glasses by superimposition of this apparent cross cylinder and actuate the Stokes' lenses accordingly.

## Revendications

1. Un photoroptère à entraînement électromoteur ayant
• au moins un élément optique tournant (rotor) dont la rotation modifie l'effet optique,
• un entraînement électromoteur pour faire tourner l'élément optique,
• une unité de commande électronique pour le réglage manuel ou commandé par ordinateur de l'effet optique voulu,
**caractérisé en ce que** l'entraînement électromoteur est constitué d'un entraînement direct au sens d'un moteur pas-à-pas et réalisé de la manière suivante:
• *n* culasses magnétisables et de conception identique sont fixées sur le rotor sur une circonférence à intervalles d'angle constants α dans un voisinage non magnétisable, la normale du cercle étant l'axe de rotation du rotor;
• *m* électro-aimants de conception identique sont montés dans un voisinage non magnétisable à des intervalles d'angle constants β sur la circonférence d'un stator, fixe dans l'espace et situé vis-à-vis du stator, la normale du cercle étant l'axe de rotation du rotor;
• la répartition est telle qu'un électro-aimant activé attire une culasse située à une distance inférieure à α/2 de lui et entraîne par là une rotation du rotor;
• les angles α et β sont choisis de telle manière qu'ils remplissent une "condition de vernier", c'est-à-dire que pour l'angle γ = |α - β|, ont a soit *m* · γ = α et (*m* + 1) · γ = β, soit *n* · γ = β et (*n* + 1) · γ = α, où *n* et *m* sont des nombres premiers ou bien il existe une condition correspondante faisant de n et / ou *m* des diviseurs entiers;
• un dispositif de commutation électronique active les électro-aimants de telle manière que l'élément optique se déplace conformément aux prescriptions de l'équipement de commande.

2. Phoroptère selon la revendication 1, **caractérisé en ce qu**'on prévoit un moyen de mesure de position pour le rotor, par exemple une barrière lumineuse, qui reconnaît au moins une position du rotor avec au moins une résolution angulaire de α/2.

3. Phoroptère selon la revendication 1, **caractérisé en ce qu**'on intègre comme éléments optiques des disques revolver destinés à recevoir des lentilles ou d'autres composants optiques, où un autre composant est amené dans l'axe optique à chaque rotation du disque revolver.

4. Phoroptère selon la revendication 1, **caractérisé en ce que** des lentilles de Stokes se trouvent dans l'axe optique en tant qu'éléments optiques et qu'elles peuvent tourner autour de celui-ci

5. Phoroptère selon la revendication 1, **caractérisé en ce que** des prismes se trouvent dans l'axe optique en tant qu'éléments optiques et qu'ils peuvent tourner autour de celui-ci.

6. Phoroptère selon la revendication 1, **caractérisé en ce que** les électro-aimants du rotor ou de deux rotors avoisinants sont montés, avec leur équipement électronique de commande, sur un module (platine).

7. Phoroptère selon la revendication 1, **caractérisé en ce qu'**on prévoit une interface électronique permettant de transmettre les valeurs de consignes nécessaires aux réglages du phoroptère émises par une unité de commande manuelle-électronique et / ou un dispositif de réfraction automatique.

8. Phoroptère selon la revendication 4, **caractérisé en ce qu**'on prévoit une unité de commande manuelle-électronique à l'aide de laquelle l'opérateur peut modifier la puissance astigmate ou l'axe astigmate des verres additionnels d'une manière qui lui paraît semblable à celle du cylindre croisé bien connu de l'homme du métier, mais où agissent en fait des moyens électroniques qui calculent cette modification des verres additionnels par superposition avec le cylindre croisé virtuel et dirigent les lentilles de Stokes en conséquence.
